# EUROPEAN PATENT SPECIFICATION

(11) **EP 4 286 022 B1**
(45) Date of publication and mention of the grant of the patent: **18.02.2026**
(21) Application number: 21923176.8
(22) Date of filing: 13.12.2021
(51) Int. Cl.: A63B 69/00, A63B 71/06, G16H 20/00, A61B 5/22, A61B 5/103, A61B 5/11, G01C 22/00, G16H 50/20, G16H 50/30, A61B 5/00, G16H 20/30, G16H 40/63, G16H 50/70

(54) **EXERCISE SUPPORT DEVICE, EXERCISE SUPPORT METHOD AND EXERCISE SUPPORT SYSTEM**
ÜBUNGSUNTERSTÜTZUNGSVORRICHTUNG, ÜBUNGSUNTERSTÜTZUNGSVERFAHREN, UND ÜBUNGSUNTERSTÜTZUNGSSYSTEM
DISPOSITIF DE PRISE EN CHARGE D'EXERCICE, PROCÉDÉ DE PRISE EN CHARGE D'EXERCICE ET SYSTÈME DE PRISE EN CHARGE D'EXERCICE

(30) Priority: 29.01.2021 JP 2021013069
(43) Date of publication of application: 06.12.2023
(73) Proprietor: OSAKA UNIVERSITY, Suita-shi Osaka 565-0871 (JP)
(72) Inventor: NAKATA, Ken, Suita-shi, Osaka 565-0871 (JP); KANAMOTO, Takashi, Suita-shi, Osaka 565-0871 (JP)
(74) Representative: DTS Patent- und Rechtsanwälte PartmbB
(86) International application number: PCT/JP2021/045783
(87) International publication number: WO 2022/163171

(56) References cited:
- WO-A1-2020/100318
- JP-A- 2009 240 404
- JP-A- 2012 135 375
- JP-A- 2014 018 312
- JP-A- 2017 159 150
- JP-A- 2020 035 140
- US-A1- 2009 171 614
- US-A1- 2018 345 081
- US-A1- 2021 254 996

## Description

### Technical Field

The present invention relates to support technology in setting an exercise menu to be continuously executed for functional recovery or capability improvement (hereinafter referred to as improvement) in rehabilitation or sports.

### Background Art

In recent years, standards for physical activity (Physical Activity: PA) directed to extend healthy life expectancy have been proposed, and the evaluation of the physical activity PA has become increasingly important (Non-Patent Literatures 1 and 2). In addition, the measurement of the physical activity PA by an acceleration sensor such as a wearable sensor device (WSD) has made remarkable progress, and usage scenes are expected to expand in the future, as a report (Non-Patent Literature 3) on the measurement of the physical activity PA by the wearable sensor device WSD for several thousands of healthy people is found, for example. On the other hand, in treatment of a patient with knee joint disease, emphasis has been on a patient-based assessment such as KOOS (Knee injury and Osteoarthritis Outcome Score), IKDC subjective score, Lysholm score.

In addition, Patent Literature 1 discloses an activity meter that calculates composite angular velocity, vertical component angular velocity, and horizontal component angular velocity from detected acceleration data in three axial directions, and, from these, performs calculation of activity intensity METs (Metabolic equivalents) and determination of a type of physical activity. Moreover, Patent Literature 2 discloses a rehabilitation support device that includes an active mass measurement unit that measures the active mass of a paralyzed-side upper limb on the basis of a detection signal from an acceleration sensor, and a display unit that displays an image showing the active mass of the paralyzed-side upper limb and an active mass target value, and, in a case in which an expected value of the activity mass in a predetermined time period is not expected to reach the active mass target value, causes the display unit to display the fact accordingly.

### Citation List

### Patent Literature

[Patent Literature 1] Japanese Patent No. 4992595 [Patent Literature 2] Japanese Unexamined Patent Application Publication No. 2020-39566

### Non-Patent Literature

[Non-Patent Literature 1] IM Lee, EJ Shiroma. Using accelerometers to measure physical activity in large-scale epidemiological studies: issues and challenges. Br J Sports Med 2014; 48: 197-201.
[Non-Patent Literature 2] KL Piercy, RP Troiano, RM Ballard et al. The Physical Activity Guidelines for Americans. JAMA 2018; 320: 2020-2028.
[Non-Patent Literature 3] EJ. Shiroma, PS. Freedson, SG. Trost et al. Patterns of Accelerometer-Assessed Sedentary Behavior in Older Women. JAMA 2013; 310: 2562-2563.
In US 2018/345081 A1 an electronic device includes a sensor, a processor, and a memory configured to store at least one instruction executed by the processor, wherein the processor is configured to collect activity information on a user related to the electronic device by using the sensor, the collecting of the activity information including creating an amount of activity of the user for a specific goal or an activity engagement level for the specific goal by using the activity information, adjust at least one of an output time point, an output cycle, the number of outputs, or the output contents of the activity guide information for the user to an activity guide parameter at least based on the amount of activity or the activity engagement level, and output the activity guide information created by using the adjusted activity guide parameter through an output device operatively connected to the processor.
In WO 2020/100318 A1 to take into account both the user's activity amount and factors other than activity amount to guide the user to have suitable lifestyle habits, this habit improving device is provided with: a first calculation unit which, for every first period, acquires an activity amount of the user totaled for each first period, and which calculates a distribution pattern that indicates the change over time in the activity amount during a second period, which includes a first period; a sorting unit which classifies multiple distribution patterns into one or more groups; a second calculation unit which, on the basis of factors other than activity amount, calculates, and associates with the groups, a target pattern as a target for the user; and a presenting unit which presents advice information to the user on the basis of the current distribution pattern and the target pattern.

### Summary of Invention

### Technical Problem

The patient-based assessment described above, in a case of enabling quantitative assessment of ADL (Activities of Daily Living: Activities of Daily Living Test) or a sports activity level by measuring the physical activity PA of a patient by using the wearable sensor device WSD, although being considered to be highly advantageous, has limitations in terms of quantitativeness, objectivity, and reproducibility, when it remains conventional.

In addition, Patent Literatures 1 and 2 do not disclose technology to support the setting of an exercise menu for functional recovery through rehabilitation or for improvement of sports ability, by use of quantitative and qualitative characteristics in each category classified according to physical activity intensity and between categories.

In view of the foregoing, the present invention proposes an exercise support apparatus, an exercise support method, an exercise support system, and a program that provide support in setting a next exercise menu for improvement to a targeted body, based on prior body motion information on a subject detected by a motion sensor.

### Solution to Problem

This problem is solved by the invention, which comprises an exercise support apparatus according to claim 1 and an exercise support method according to claim 6. The dependent claims define further preferred embodiments. An exercise support apparatus according to one aspect of the present invention includes an information processing unit that supports a setting of a next exercise menu for improvement to a targeted body, based on prior body motion information on a subject detected by a motion sensor, and the information processing unit includes a target information processing means, a measurement information processing means, and an output processing means. The target information processing means outputs distribution information on an amount of activity and a number of steps of at least one category obtained by classifying activity intensity that has been obtained from a target people group in advance, stored in a storage unit, and selected in advance, to an output unit. The measurement information processing unit calculates the amount of activity and the number of steps for the same category as a selected category, based on body motion information on the subject, and that stores a calculation result in a measurement data storage unit. The output processing means outputs the amount of activity and the number of steps that are stored in the measurement data storage unit, to the output unit.

In addition, an exercise support system according to the present invention includes the exercise support apparatus, and a motion sensor that detects the prior body motion information on the subject and sends a detection result to the information processing unit.

Moreover, an exercise support method according to the present invention supports a setting of a next exercise menu for improvement to a targeted body, based on prior body motion information on a subject detected by a motion sensor, and includes a step of outputting distribution information on an amount of activity and a number of steps of at least one category obtained by classifying activity intensity that has been obtained from a target people group in advance, stored in a storage unit, and selected in advance, to an output unit, a step of calculating the amount of activity and the number of steps for a same category as a selected category, based on body motion information on the subject, and that stores a calculation result in a measurement data storage unit, and a step of outputting the amount of activity and the number of steps that are stored in the measurement data storage unit, to the output unit.

Furthermore, a program according to the present invention uses a computer to support a setting of a next exercise menu for improvement to a targeted body, based on prior body motion information on a subject detected by a motion sensor, and causes the computer to execute outputting distribution information on an amount of activity and a number of steps of at least one category obtained by classifying activity intensity that has been obtained from a target people group in advance, stored in a storage unit, and selected in advance, to an output unit, calculating the amount of activity and the number of steps for a same category as a selected category, based on body motion information on the subject, and that stores a calculation result in a measurement data storage unit, and outputting the amount of activity and the number of steps that are stored in the measurement data storage unit, to the output unit.

According to these inventions, the distribution information on the amount of activity and the number of steps of at least one category obtained by classifying activity intensity that has been obtained from a target people group in advance, stored in a storage unit, and selected in advance, is outputted to an output unit. In addition, the amount of activity and the number of steps for a same category as a selected category, based on body motion information on the subject is calculated and a calculation result is stored in a measurement data storage unit. Subsequently, the amount of activity and the number of steps of the subject that are stored in the measurement data storage unit are outputted to the output unit. Therefore, since it is possible to recognize the distribution information on the target people group, it becomes possible to provide suitable support in setting the next exercise menu for improvement to the targeted body, based on the prior body motion information on the subject detected by the motion sensor. In the above, the subject and the target people group are defined such that, in a case in which the subject is a patient with a disease of a leg, that is, a knee joint, for example, or a patient with sports injury, the target people group may be assumed to be a group of healthy people, and, in a case in which the subject is an athlete, the target group may be typically assumed to be a group of top athletes in the field.

### Advantageous Effects of the Disclosure

According to the present invention, support in setting the next exercise menu for improvement to a target physical state is more accurately performed. Furthermore, according to the present invention, the storage unit stores information of at least one category selected among a plurality of categories while the measurement data storage unit outputs information of the same category as the category stored in the storage unit, so that the overall storage capacity is reduced, and output processing of the information is reduced.

### Brief Description of Drawings

FIG. 1 is a block diagram showing one embodiment of an exercise support system according to the present invention.
FIG. 2 shows a chart showing a relationship between activity intensity, a category, activity content of the category, and a subcategory, and also showing a relationship with a separate evaluation.
FIG. 3A and FIG. 3B show a distribution state being a result of an experiment of an amount of activity and the number of steps of a healthy group and a patient group, on a coordinate system of the amount of activity and the number of steps of each category of the activity intensity, FIG. 3A shows the SED category of the lowest activity intensity, and FIG. 3B shows the LPA category of the next lesser intensity.
FIG. 4A, FIG. 4B, and FIG. 4C show a distribution state being a result of an experiment of an amount of activity and the number of steps of a healthy group and a patient group, FIG. 4A is a view showing an MVPA category of high activity intensity, FIG. 4B is a view showing a lower MPA category obtained by subdividing the MVPA category, and FIG. 4C is a view showing a subdivided VPA category of higher intensity.
FIG. 5 shows a distribution state being a result of an experiment of an amount of activity and the number of steps of a healthy group and a patient group, and is a view of Long-bout MVPA showing the amount of activity that continues for a predetermined long period of time, in the MVPA category.
FIG. 6 is a table showing the average of the amount of activity and the number of steps in the bottom line for the healthy group and the patient group in each category in the present experiment, as well as each correlation value p.
FIG. 7 is a sample graph illustrating an example of a rehabilitation process of a patient.
FIG. 8 is a flow chart showing an example of measurement data processing that a CPU of a control unit executes.
FIG. 9 is a flow chart showing an example of rehabilitation support processing that the CPU of the control unit executes.

### Description of Embodiments

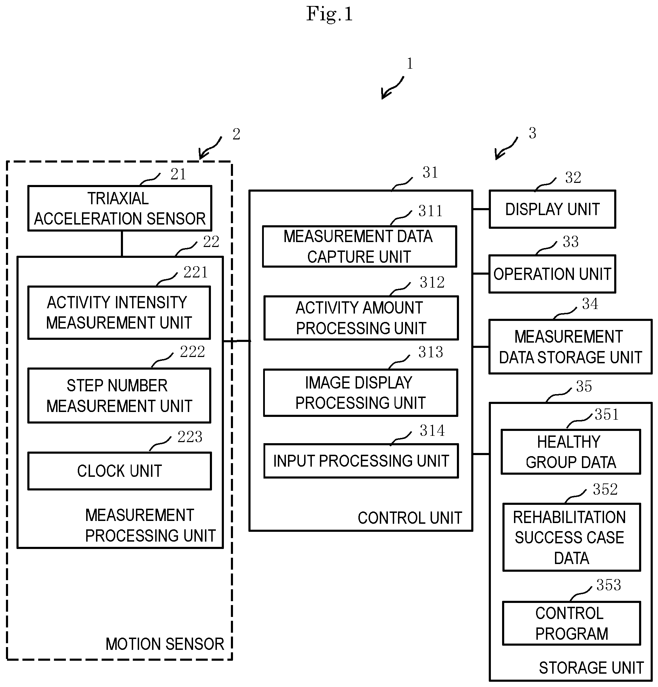

FIG. 1 is a block diagram showing one embodiment of an exercise support system 1 according to the present invention. The exercise support system 1 includes a motion sensor 2 and an information processing apparatus 3 that, in the present embodiment, are able to communicate information to and from each other by wired or wireless communication means.

The motion sensor 2 is attached to a human body and detects a motion of the human body, especially the acceleration of the motion of a human body, and, in the present embodiment, a triaxial acceleration sensor 21 is employed and is integrally configured with a measurement processing unit 22. As an acceleration sensor, a capacitance type and a piezoelectric type are able to be employed, and various acceleration sensors based on other detection principles may be used. The motion sensor 2 includes an attachment tool such as a clip or a fastening tool that is not shown, and is used by being attached to a central part of the human body, preferably a suitable position around a waist, through such an attachment tool. The motion sensor 2 is attachable around a waist so that the triaxial acceleration sensor faces up and down, as well as forward and backward, and left and right to the human body, and detects each direction component from the acceleration sensor corresponding to each axis.

The motion sensor 2 includes a not-shown power switch, and is activated to perform a detection operation while the switch is on. In an embodiment of rehabilitation support (exercise support for rehabilitation) for knee joint disease to be described below, the sensor is activated for a preset predetermined period of time between waking and sleeping, for example, about 10 hours, and detects daily movements of the human body as well as rehabilitation movements to be described below. It is to be noted that the predetermined time may be set by specifying a start time and an end time or may also be a flexible setting of a time period between waking and sleeping, literally, for example. Alternatively, data may be collected for a longer period of time along with time information, and, when the data is captured, data for a required period of time may be selectively captured.

The configuration and function of the triaxial acceleration sensor 21 and the measurement processing unit 22 are able to employ the same technology as the technology disclosed in Patent Literature 1 as an example. Hereinafter, in a brief description, the measurement processing unit 22 includes a processor (a CPU), and, by executing a measurement program stored in a storage unit 23, functions as an activity intensity measurement unit 221, a step number measurement unit 222, and a clock unit 223 that clocks time or required time.

The activity intensity measurement unit 221 captures acceleration data detected by the triaxial acceleration sensor 21 at a predetermined period of about several tens of Hz, for example, and measures activity intensity from the captured time-series acceleration data. For example, synthetic acceleration S, vertical component acceleration Sv, and horizontal component acceleration Sh are calculated from the triaxial acceleration data, and, by using these as determination conditions, for example, the level of the synthetic acceleration S and the ratio of the component acceleration Sv and Sh, the activity intensity (METs: Metabolic equivalents) is calculated from the synthetic acceleration, and the type of physical activity such as life activity, exercise, or rest, for example, is also classified. In addition, the activity intensity measurement unit 221 calculates the activity intensity METs from the detected acceleration as data for each unit time (1 minute, for example).

The step number measurement unit 222 measures (counts) the number of times the detected acceleration of the vertical component exceeds a predetermined threshold value, and defines the number as the cumulative number of steps within a detection period.

The information processing apparatus 3 includes a control unit 31 configured by a processor (a CPU). The control unit 31 is connected to a display unit 32 that displays an image, an operation unit 33 that receives operating instructions from the outside, a measurement data storage unit 34, and a storage unit 35. It is to be noted that the operation unit 33 may be configured by a touch panel obtained by stacking a transparent pressure-sensitive panel element on the display unit 32.

The measurement data storage unit 34 is data measured by the motion sensor 2, and stores the data captured by the information processing apparatus 3 for each patient ID. The storage unit 35 has a healthy group data storage unit 351, a rehabilitation success case data storage unit 352, and a control program data storage unit 353. Healthy group data refers to activity content detected in the same period (10 hours between waking and sleeping in the above example) as a patient, and will be described in detail below. In addition, the storage unit 35 includes a work area (a main memory unit) that executes information processing, in addition to a memory area.

The control unit 31, by reading and executing a control program from the control program data storage unit 353 to the work area, functions as a measurement data capture unit 311, an activity amount processing unit 312, an image display processing unit 313, and an input processing unit 314 that processes reception of various types of information to be inputted through the operation unit 33 and capture to the storage unit.

The measurement data capture unit 311, according to measurement data capture instructions from the information processing apparatus 3, captures to the measurement data storage unit 34 in advance measurement data of the physical activity and the number of steps of a patient with knee joint disease that have been measured by the motion sensor 2 for a predetermined time of the previous time, for example.

The activity amount processing unit 312 divides (classifies) the captured measurement data of a patient into each category that is set in advance according to activity intensity, and performs processing to calculate an amount of activity Ex for each category.

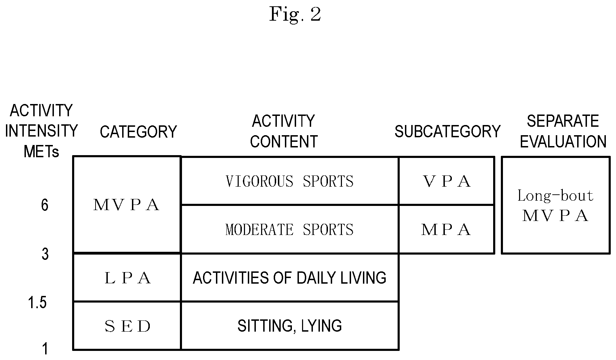

FIG. 2 shows a chart showing each of a relationship between activity intensity, a category, activity content of the category, and a subcategory, and also showing a relationship with a separate evaluation. In FIG. 2, the physical activity PA, according to METs of the activity intensity, is classified to a plurality of categories of MVPA including SED (Sedentary: 1 to 1.5 METs) corresponding to a sitting and lying state, LPA (Light Physical Activity: 1.6 to 2.9 METs) corresponding to activities of daily living, MPA (Moderate Physical Activity: 3.0 to 5.9 METs) corresponding to further light sports, and VPA (Vigorous Physical Activity: ≥ 6.0 METs) corresponding to intense sports. In addition, as a guideline for health promotion, long-bout MVPA for a separate evaluation that continuously measures an amount of activity Ex included in the MVPA for at least 10 minutes is further employed.

The activity amount processing unit 312 calculates the amount of activity Ex by accumulating the activity intensity METs data corresponding to each category per unit time, for example, one minute. Moreover, the activity amount processing unit 312 calculates the amount of activity Ex for Long-bout MVPA for a separate evaluation. A calculation result is stored in the measurement data storage unit 34 when necessary.

Next, a physical activity experiment was conducted on a group of healthy subjects (a healthy group) and a group of patients (a patient group), and not only a quantitative analysis but also a qualitative analysis was conducted to the result. The analysis, as shown below, was conducted by use of (1) comparison between the patient group and the healthy group regarding the amount of activity Ex and the number of steps in each category of the activity intensity METs, and (2) Publicly known Student's t test and Pearson's correlation coefficient for the purpose of examining a relationship between the amount of activity Ex and the number of steps within a category. It is to be noted that, in the present analysis, a significance level at a P value of correlation evaluation was set at a general 5%.

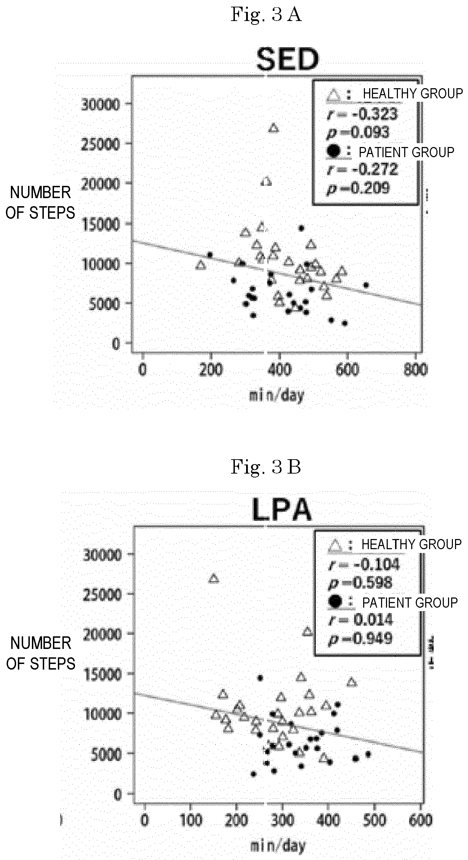

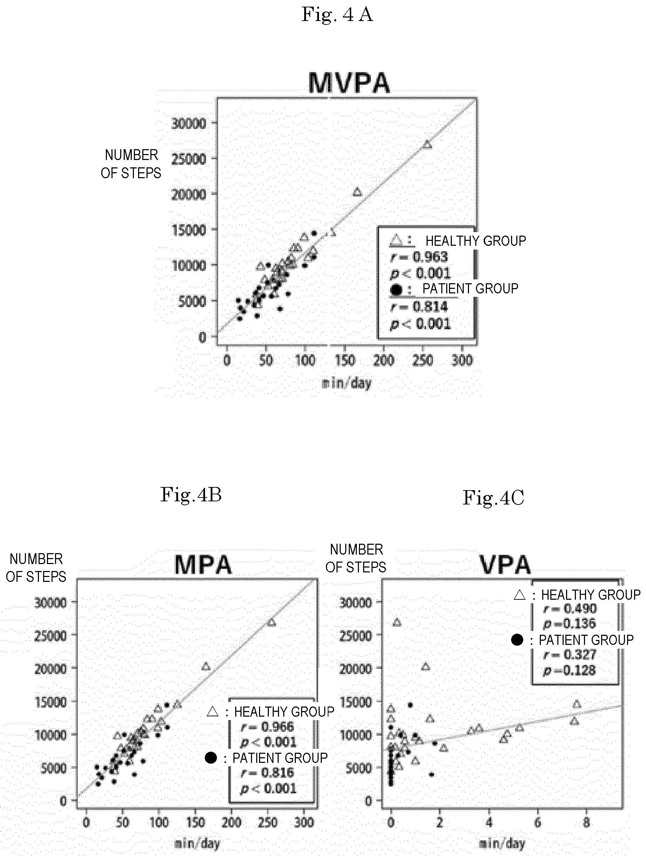

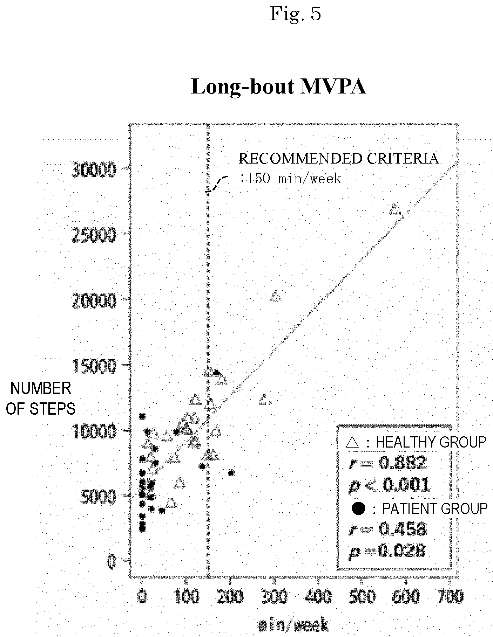

FIG. 3A to FIG. 5 show a distribution state being the result of the experiment to obtain data on the amount of activity Ex and the number of steps for the healthy group and the patient group, in a coordinate system of the amount of activity Ex and the number of steps in each category of the activity intensity METs. FIG. 3A shows the SED category of the lowest activity intensity, and FIG. 3B shows the LPA category of the next lesser intensity. In addition, FIG. 4A shows an MVPA category of high activity intensity, FIG. 4B shows a lower MPA category obtained by subdividing the MVPA category, and FIG. 4C shows a subdivided VPA category of higher intensity. Moreover, FIG. 5 shows the Long-bout MVPA in the MVPA category.

The experimental result data shown in FIG. 3A to FIG. 5, and FIG. 6 includes a patient with knee joint disease as a subject. The subjects of the present experiment were 23 outpatients (a patient group: 10 males and 13 females, aged 22 to 81 years (mean age 51 years)) attending Osaka University Hospital and 28 healthy people (a healthy group: 12 males, 16 females, aged 18 to 65 years (mean age 28 years)) with no history of knee joint disease, and measurement information obtained by wearing the motion sensor 2 (an activity meter: Active Style Pro HJA-750C, OMRON Healthcare, Japan) on the lower back for 10 hours or more per day for 7 consecutive days for both groups was summarized as in FIG. 3A to FIG. 6.

In addition, from the detection result of a triaxial accelerometer of the activity meter applied to the present experiment, a value of Metabolic equivalents (METs) for one minute is extracted by use of the "Activity Meter Application" attached to the activity meter and used for PA evaluation, based on an algorithm or the like disclosed in Patent Literature 1 or a reference material (K Ohkawara, Y Oshima, Y Hikihara et al. Real-time estimation of daily physical activity intensity by a triaxial accelerometer and a gravity-removal classification algorithm. Br J Nutr 2011; 105: 1681-1691., and Y Oshima, K Kawaguchi, S Tanaka et al. Classifying household and locomotive activities using a triaxial accelerometer. Gait Posture 2010; 31: 370-374.) At the same time, information on the number of steps was also extracted.

The amount of activity for each person is added up for each category. In the drawing, an open triangle mark indicates a coordinate position of the amount of activity and the number of steps for each person in the group of healthy people (the healthy group), and a black circle mark indicates a coordinate position of the amount of activity and the number of steps for each person in the patient group. It is to be noted that the data in the present experiment includes no exercise according to the instructions of the exercise menu for both healthy people and patients. For the distribution in each drawing, r value indicates a correlation coefficient and, P value indicates a correlation value, and a straight line indicates a regression line of the healthy group.

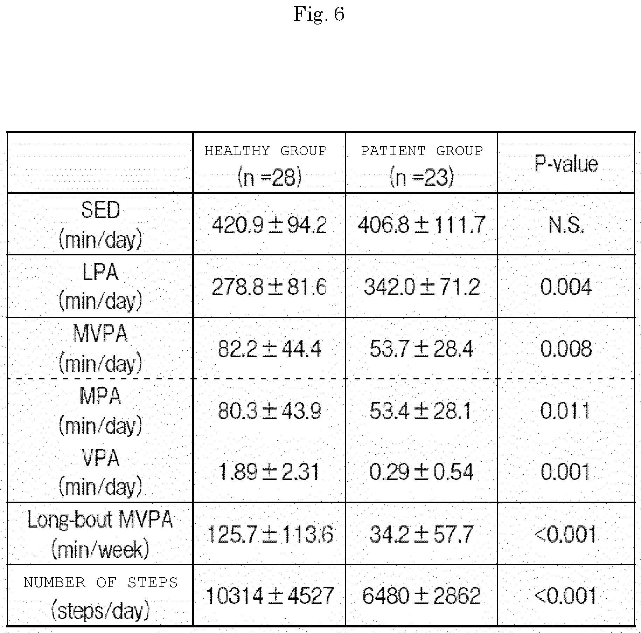

Next, FIG. 3A to FIG. 6 will be analyzed. In comparison with the amount of activity Ex and the number of steps in both groups, the patient group had significantly lower values for MVPA (FIG. 4A), long-bout MVPA (FIG. 5), and the number of steps (see the bottom line of FIG. 6), while having a higher value for LPA (FIG. 3B). Although SED and LPA showed no significant differences between the two groups, the patient group had a slightly lower value in the number of steps (FIG. 3A). The patient group had a lower value in both MPA and VPA (FIG. 4B and FIG. 4C) that are obtained by subdividing MVPA.

Regarding the correlation between the amount of activity Ex and the number of steps, while SED and LPA in FIG. 3A and FIG. 3B showed no significant correlation, MVPA showed a strong positive correlation in both groups (r = 0.963, r = 0.814) (FIG. 4A). In addition, in a case in which MPA and VPA are divided, as shown in FIG. 4B and FIG. 4C, although a similarly strong positive correlation with MPA (r = 0.966 and r = 0.816) was found, a positive correlation with VPA was weak (r = 0.490).

Moreover, regarding Long-bout MVPA, as shown in FIG. 5, as compared with the healthy group (r = 0.882), a lower correlation resulted in the patient group (r =0.458), and the amount of activity was also low in the patient group, regardless of the number of steps, and only two patients (8.7%) satisfied the recommended criteria of 150 minutes/week or more, as compared with eight persons (28.6%) in the healthy group, which showed a difference between the two groups.

Next, FIG. 6 is a table showing the average of the amount of activity and the number of steps shown in the bottom line for the healthy group and the patient group in each category in the present experiment, as well as each correlation value p. In the present experiment, in activity amount evaluation by the motion sensor 2, a result that the patient group was less active in the MPA, VPA, and long-bout MVPA categories was obtained. Such classification in a direction of intensity based on the measurement result from the motion sensor 2 allowed quantitative evaluation of the amount of physical activity within a category and qualitative evaluation between categories. Through these, the evaluation of the amount of physical activity including the categories of SED, LPA, and MVPA, and even the categories from MPA, VPA up to Long-bout MVPA, shows respective characteristics differences between the two groups, and the utilization of these differences is expected to be a powerful evaluation tool in a case of supporting therapeutic intervention (rehabilitation) and evaluating the effect of intervention.

In other words, conditions of a category for each of the categories to be applicable as a tool for evaluating rehabilitation support and the effectiveness are preferably that the distribution of the healthy group and the distribution of the patient group are significantly separated (different), and that a correlation is observed in the distribution of the healthy group. A condition for the applicable category (a category to be selected for support) is that the amount of activity and the number of steps in the initial phase of rehabilitation in the patient and the distribution information on the healthy group that is the target of recovery have a significant difference. With use of the existence of such a significant difference from the healthy group, a measurement result position of a patient during rehabilitation on a coordinate system and the distribution of the healthy group as shown in FIG. 3A to FIG. 5 are displayed, and a positional relationship between the two (the healthy group and the patient) is able to be easily and visibly presented, which makes it possible to support a gradual setting by a healthcare worker of exercise menu to intervene in a patient, based on the positional relationship or the like. The exercise menu includes exercise content (type), exercise intensity, and exercise duration. In addition, since the daily activity content of a patient is also included in the measurement result, a setting of the next exercise menu based on a comprehensive understanding of the daily activity content is accurate and easy.

With effective support in such a gradual setting, it is possible to provide accurate exercise support that gradually approaches from the outside of the distribution of the healthy group toward the distribution and eventually leads into the distribution effectively, that is, to a smooth recovery. It is to be noted that the categories used for contrast may be all the categories, focusing on some significant differences or may be narrowed down to at least one or more predetermined categories, utilizing more significant differences.

Returning to FIG. 1, the image display processing unit 313 displays image information on the display unit 32. In other words, the image display processing unit 313 displays the distribution information on at least one or more predetermined categories of the healthy group used for the contrast as an image on the coordinate system, along with the display of the image information developed on the coordinate system from the measurement data for a predetermined time period immediately before the patient, herein for 10 hours, processed by the activity amount processing unit 312. It is to be noted that the category that satisfies the condition may be fixed or may be appropriately switched depending on the level of recovery associated with rehabilitation.

The image information on the patient and the healthy group may be displayed on an image of each coordinate system or may be superimposed and displayed on an image of a shared coordinate system. The image information of the amount of activity of the patient may display data for all categories or may be only the category corresponding to the category on a healthy group side. It is to be noted that, in an aspect of superimposing and displaying on an image of the same coordinate system, a display mark on a patient side is preferably displayed differently from a display mode on the healthy group side, in shape, size, blinking or no blinking, color, or the like, so as to be identifiable. In a case of being shared and displayed, the measurement data position of the patient with respect to the distribution of the healthy group is displayed so as to be relatively easily identified.

The rehabilitation success case data storage unit 352 stores at least one or more past successful cases in which knee joint disease was suitably recovered by rehabilitation, including gradual rehabilitation time points and the exercise menu that was set at each time point, as well as further the measurement data of the patient in each case. The input processing unit 314, among rehabilitation success case data, when necessary, extracts measurement data similar to the measurement data of a patient, and a similar successful case data at a rehabilitation time point, and causes the display unit 32 to display the data by the image display processing unit 313, automatically, or through the operation unit 33 to provide support of a setting of the exercise menu.

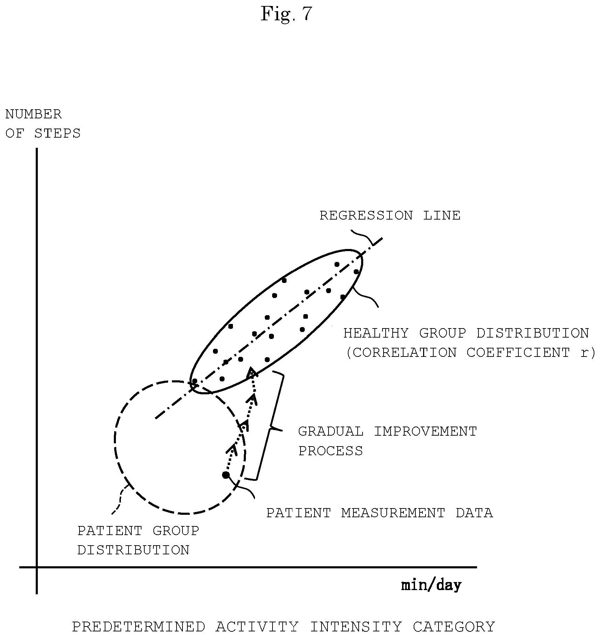

Herein, an example of rehabilitation improvement history of a patient will be illustrated in a sample graph of FIG. 7. FIG. 7 illustrates an image of the rehabilitation support and shows a process of rehabilitation improvement of one patient in a certain category (MVPA, for example). The coordinate position of the patient moves from a position of patient measurement data present in the patient group distribution at the beginning of rehabilitation toward the distribution of the healthy group or gradually approaches (indicated by each dashed arrow in FIG. 7) the distribution of the healthy group by performing a set exercise menu for rehabilitation every day over a plurality of days, and finally comes to be in the distribution of the healthy group, which indicates that the patient improves and recovers. As shown in FIG. 7, the history of the gradual measurement data of the patient is superimposed and displayed, so that the process of recovery is able to be checked, and accurate setting of the next exercise menu is expected.

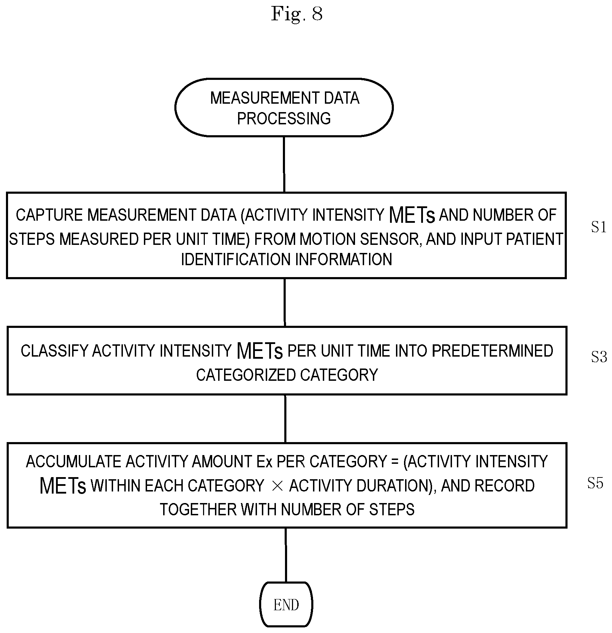

FIG. 8 is a flow chart showing an example of measurement data processing that the processor (the CPU) of the control unit 31 executes. First, the measurement data (the activity intensity METs measured per unit time, the number of steps) of the patient is captured from the motion sensor 2 and the identification information on the patient is inputted from the motion sensor 2 or from the operation unit 33 (Step S1). Next, the measurement data of the activity intensity METs per unit time is classified (sorted) into each category (Step S3). Subsequently, the amount of activity Ex for each category is accumulated with the activity intensity METs x activity time within each category, and is stored in the measurement data storage unit 34 together with the number of steps (Step S5).

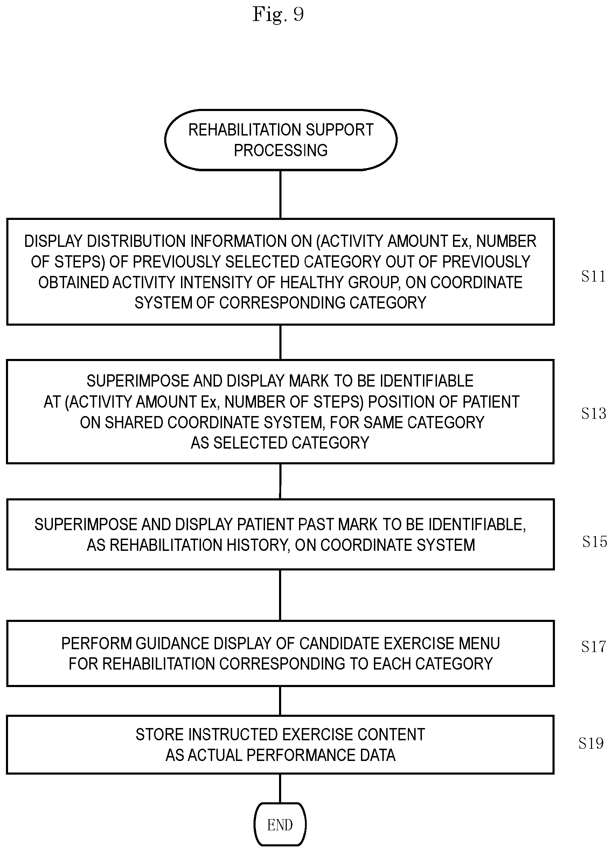

FIG. 9 is a flow chart showing an example of rehabilitation support processing that the processor (the CPU) of the control unit 31 executes. First, the image display processing unit 313, among the previously obtained activity intensity of the healthy group, displays the distribution information on (the activity amount Ex, the number of steps) of a previously selected category having information that is able to be significantly contrasted with the data of the patient on the coordinate system of a corresponding category (Step S11). Next, the image display processing unit 313 superimposes and displays a mark at a coordinate position of the data (the amount of activity Ex, the number of steps) obtained from the patient side on the shared coordinate system so as to be identifiable from each mark on the healthy group side, for the same category as the selected category (Step S13). In addition, the image display processing unit 313 automatically, through operating instructions, or the like, superimposes and displays the mark at a past data position of the patient to be identifiable as rehabilitation history, on the same coordinate system (Step S15). Furthermore, guidance display of candidate exercise menu for rehabilitation is performed at a position corresponding to each category, through operating instructions when necessary (Step S17). Alternatively, the rehabilitation success case data may be displayed. Then, the input processing unit 314 records the exercise content for rehabilitation that is set by a healthcare worker, corresponding to the patient ID, in the measurement data storage unit 34, for example (Step S19).

In the present invention, although the group of healthy people being a target is set uniformly, the healthy people may be divided into age groups, gender, or the like, to create the healthy group data, and data for contrast that is closer to the attributes of the patient may be used. In addition, the present invention may include longitudinal evaluation of the process of treatment (rehabilitation) of a patient with knee joint disease from a postoperative period, and may also be applicable further to the evaluation and guidance of the effectiveness of treatment of sports injury, rehabilitation, and return to sports.

The present invention may provide the measurement processing unit 22 near the information processing apparatus 3, and, conversely, may provide the activity amount processing unit 312 near the motion sensor 2. In addition, the output of patient side data and the distribution information on the healthy group may be printed out by a printer, instead of the display unit 32 that shows an image.

Moreover, the setting of the exercise content for rehabilitation may be set to the same exercise content for one day or for several days, and the measurement data may be collectively captured and evaluated.

In addition, the communication between the motion sensor 2 on the patient side and the information processing unit 3 on a hospital side may use Internet environment between the home and the hospital, typically, such as a WAN, in addition to short-range communication.

In addition, in the present invention, data of a list of the exercise menu may be included in the storage unit 35 of the information processing apparatus 3. The exercise menu list is preferably classified corresponding to each category of activity intensity, for example, and preferably includes an exercise type, as well as exercise intensity and exercise duration. The exercise menu list is able to provide support in setting the exercise menu, for example, automatically or by receiving operation instructions from the operation unit 33 and displaying the list by the image display processing unit 313 at an appropriate position on the display unit 32 so as to be referable for each corresponding category, for example, as appropriate.

As described above, the exercise support apparatus according to the present invention includes an information processing unit that supports a setting of a next exercise menu for improvement to a targeted body, based on prior body motion information on a subject detected by a motion sensor, and the information processing unit includes the following target information processing means, measurement information processing means, and output processing means. The target information processing means outputs distribution information on an amount of activity and a number of steps of at least one category obtained by classifying activity intensity that has been obtained from the target people group in advance, stored in a storage unit, and selected in, to an output unit. The measurement information processing unit calculates the amount of activity and the number of steps for the same category as a selected category, based on body motion information on the subject, and that stores a calculation result in a measurement data storage unit. The output processing means outputs the amount of activity and the number of steps that are stored in the measurement data storage unit, to the output unit.

In addition, the exercise support system according to the present invention preferably includes the exercise support apparatus, and a motion sensor that detects the prior body motion information on the subject and sends a detection result to the information processing unit.

In addition, the exercise support method according to the present invention preferably supports a setting of a next exercise menu for improvement to a targeted body, based on prior body motion information on a subject detected by a motion sensor, and preferably includes a step of outputting distribution information on an amount of activity and a number of steps of at least one category obtained by classifying activity intensity that has been obtained from a target people group in advance, stored in a storage unit, and selected in advance, to an output unit, a step of calculating the amount of activity and the number of steps for a same category as a selected category, based on body motion information on the subject, and that stores a calculation result in a measurement data storage unit, and a step of outputting the amount of activity and the number of steps that are stored in the measurement data storage unit, to the output unit.

In addition, the non-transitory computer readable storage medium storing the program according to the present invention that preferably uses a computer to support a setting of a next exercise menu for improvement to a targeted body, based on prior body motion information on a subject detected by a motion sensor, the program preferably causing the computer to execute outputting distribution information on an amount of activity and a number of steps of at least one category obtained by classifying activity intensity that has been obtained from a target people group in advance, stored in a storage unit, and selected in advance, to an output unit, calculating the amount of activity and the number of steps for a same category as a selected category, based on body motion information on the_subject, and that stores a calculation result in a measurement data storage unit, and outputting the amount of activity and the number of steps that are stored in the measurement data storage unit, to the output unit.

According to these inventions, the distribution information on the amount of activity and the number of steps of at least one category obtained by classifying activity intensity that has been obtained from a target people group in advance, stored in a storage unit, and selected in advance, is outputted-to an output unit. In addition, the amount of activity and the number of steps for a same category as a selected category, based on body motion information on the subject is calculated and a calculation result is stored in a measurement data storage unit. Subsequently, the amount of activity and the number of steps of the subject that are stored in the measurement data storage unit are outputted to the output unit. Therefore, since it is possible to recognize the distribution information on the target people group, it becomes possible to provide suitable support in setting the next exercise menu for improvement to the targeted body, based on the prior body motion information on the subject detected by the motion sensor. In the above, the subject and the target people group are defined such that, in a case in which the subject is a patient with a disease of a leg, that is, a knee joint, for example, or a patient with sports injury, the target people group may be assumed to be a group of healthy people, and, in a case in which the subject is an athlete, the target group may be typically assumed to be a group of top athletes in the field.

In addition, the output unit is preferably a display unit that displays an image. According to this configuration, the measurement data of the subject and the distribution information on the target people group are displayed in the image.

In addition, the output processing means preferably displays the amount of activity and the number of steps that have been calculated by the measurement information processing means, on a coordinate system of the selected each category. According to this configuration, since both pieces of information are displayed on a similar coordinate system, identification becomes easy.

In addition, the present invention preferably superimposes and displays the amount of activity and the number of steps that have been calculated by the measurement information processing means so as to be identifiable, on the shared coordinate system of each category displayed on the display unit. According to this configuration, since both pieces of information are superimposed and displayed on the shared coordinate system, identification of the both becomes easier and more accurate.

In addition, the selected category is preferably a category with a significant difference between the amount of activity and the number of steps of the subject in an initial period of support and the distribution information on the target people group. According to this configuration, since a difference is able to be more recognized in the early stage of support, for example, at the beginning of rehabilitation, effective support is able to be provided, for example, when setting an exercise menu for rehabilitation.

In addition, the selected category is preferably a category in which the distribution information on the target people group shows relatively high correlation. According to this configuration, it is possible to support the subject to improve more accurately to the physical condition of the target person.

In addition, the detection of the body motion information is preferably performed at a preset time between waking and sleeping. According to this configuration, the overall exercise condition also including daily life activities is able to be grasped, and a setting of the next exercise menu is able to be provided.

In addition, the distribution information is preferably at least one of all values of each of the amount of activity and the number of steps of the target people group, and a regression line to distribution of a value of each of the amount of activity and the number of steps of the target people group. According to this configuration, it is possible to output information on the target people group in an appropriate manner, which facilitates recognition of a difference.

In the present disclosure, the subject is preferably a patient with knee joint disease, and the target people group is preferably a healthy people group. According to this configuration, effective support is able to be provided for a setting of the exercise menu for rehabilitation to the patient with knee joint disease.

### Reference Signs List

1 exercise support system
2 motion sensors
21 triaxial acceleration sensor
22 measurement processing unit
3 information processing apparatus (exercise support apparatus)
31 control unit (information processing unit)
312 activity amount processing unit (measurement information processing means)
313 image display processing unit (target information processing means, output processing means)
32 display unit (output unit)
33 operation unit
34 measurement data storage unit
35 storage unit
351 healthy group data storage unit

## Claims

1. An exercise support apparatus (3) that supports a setting of an exercise menu for improvement to a targeted body, based on prior body motion information on a subject detected by a motion sensor (2), wherein the exercise support apparatus (3) comprises:
a first healthy group data storage unit (351) configured to store distribution information on an amount of activity and a number of steps of at least one category classified by activity intensity and selected in advance, the distribution information for the one category being obtained for a specified period in advance from a target people group;
an activity amount processing unit (312) configured to calculate the amount of activity and the number of steps of a same category as the selected category, based on body motion information on the subject, the motion information being detected by the motion sensor (2) for a same period as the specified period,
a measurement data storage unit (34) configured to store results calculated each time by the activity amount processing unit (312); and
a display unit (32) configured to display an image;
an output processing means (313), configured to read the distribution information stored in the healthy group data storage unit (351) to the display unit (32), and to display the distribution information with an amount of activity and a number of steps as coordinate axes, after the activity amount processing unit (312) stored the results, and to read
the amount of activity and the number of steps stored in the measurement data storage unit (34) on the subject for each time to the display unit (32) as information of gradual position marks, and to display them with the shared coordinate axes to which the distribution information is superimposed; and an input processing unit (314) configured to receive a next setting of the exercise menu while the information of gradual position marks is superimposed on the distribution information with the same coordinate axes by the display unit (32).

2. The exercise support apparatus (3) according to claim 1, wherein the distribution information is information of position marks of the amount of activity and the number of steps on each subject of the target people group.

3. The exercise support apparatus (3) according to claim 1 or claim 2, wherein the specified period is a period which is preset between waking and sleeping.

4. An exercise support system comprising the exercise support apparatus according to any of claims 1 to 3, and the motion sensor (2) configured to detect the body motion information on the subject.

5. The exercise support system according to claim 4, wherein communication between the exercise support apparatus (3) and the motion sensor (2) can be communicated by either wired or wireless means of communication.

6. An exercise support method that supports a setting of a next exercise menu for improvement to a targeted body, based on prior body motion information on a subject detected by a motion sensor (2), wherein the exercise support method comprises:
a step of storing distribution information on an amount of activity and a number of steps of at least one category classified by activity intensity and selected in advance into a healthy group data storage unit (351), the distribution information for the one category being obtained for a specified period in advance from a target people group;
a step of calculating the amount of activity and the number of steps for a same category as the selected category, based on body motion information on the subject, the motion information being detected by the motion sensor (2) for a same period as the specified period;
a step of storing results each time calculated by the step of calculating into a measurement data storage unit (34); and
a step of reading the distribution information stored in the healthy group data storage unit (351) to the display unit (32), and displaying the distribution information with an amount of activity and a number of steps as coordinate axes, after the results are stored into the measurement data storage unit (34),
reading the amount of activity and the number of steps stored in the measurement data storage unit (34) on the subject for each time to the display unit (32) as information of gradual position marks, and displaying them with the shared coordinate axes to which the distribution information is superimposed; and
a step of receiving a next setting of the exercise menu while the information of gradual position marks is superimposed on the distribution information with the same coordinate axes by the display unit (32).

## Patentansprüche

1. Übungsunterstützungsvorrichtung (3), die auf der Grundlage von früheren Körperbewegungsinformationen über einen Probanden, die von einem Bewegungssensor (2) erfasst wurden, eine Festlegung eines Übungsmenüs zur Verbesserung eines Zielkörpers unterstützt, wobei
die Übungsunterstützungsvorrichtung (3) aufweist:
eine ersten Gesundheitsgruppendaten-Speichereinheit (351), die dafür ausgelegt ist, Verteilungsinformationen über einen Aktivitätsumfang und eine Anzahl von Schritten von zumindest einer nach Aktivitätsintensität klassifizierten und vorab ausgewählten Kategorie zu speichern, wobei die Verteilungsinformationen für die eine Kategorie vorab von einer Zielpersonengruppe für einen bestimmten Zeitraum erhalten werden;
eine Aktivitätsumfangs-Verarbeitungseinheit (312), die dafür ausgelegt ist, den Aktivitätsumfang und die Anzahl von Schritten derselben Kategorie wie die ausgewählte Kategorie auf der Grundlage von Körperbewegungsinformationen über den Probanden zu berechnen, wobei die Bewegungsinformationen durch den Bewegungssensor (2) für denselben Zeitraum wie der bestimmte Zeitraum erfasst werden,
eine Messdaten-Speichereinheit (34), die dafür ausgelegt ist, Ergebnisse zu speichern, die jeweils von der Aktivitätsumfangs-Verarbeitungseinheit (312) berechnet werden; und
eine Anzeigeeinheit (32), die dafür ausgelegt ist, ein Bild anzuzeigen;
ein Ausgabeverarbeitungsmittel (313), das dafür ausgelegt ist, die in der Gesundheitsgruppendaten-Speichereinheit (351) gespeicherten Verteilungsinformationen in die Anzeigeeinheit (32) einzulesen und die Verteilungsinformationen mit einem Aktivitätsumfang und einer Anzahl von Schritten als Koordinatenachsen anzuzeigen, nachdem die Aktivitätsumfangs-Verarbeitungseinheit (312) die Ergebnisse gespeichert hat, und
den Aktivitätsumfang und die Anzahl von Schritten, die in der Messdaten-Speichereinheit (34) über den Probanden gespeichert sind, jeweils in die Anzeigeeinheit (32) als Informationen gradueller Positionsmarkierungen einzulesen, und
sie mit den mitgeteilten Koordinatenachsen, denen die Verteilungsinformationen überlagert wurden, anzuzeigen; und
eine Eingabeverarbeitungseinheit (314), die dafür ausgelegt ist, die nächste Festlegung des Übungsmenüs zu empfangen, während die Informationen gradueller Positionsmarkierungen durch die Anzeigeeinheit (32) den Verteilungsinformationen mit denselben Koordinatenachsen überlagert werden.

2. Übungsunterstützungsvorrichtung (3) nach Anspruch 1, wobei es sich bei den Verteilungsinformationen um Informationen über Positionsmarkierungen des Aktivitätsumfangs und die Anzahl von Schritten von jedem Probanden der Zielpersonengruppe handelt.

3. Übungsunterstützungsvorrichtung (3) nach Anspruch 1 oder Anspruch 2, wobei es sich bei dem bestimmten Zeitraum um einen Zeitraum handelt, der zwischen Wachsein und Schlafen voreingestellt ist.

4. Übungsunterstützungssystem, die Übungsunterstützungsvorrichtung nach einem der Ansprüche 1 bis 3 und den Bewegungssensor (2) aufweisend, der dafür ausgelegt ist, die Körperbewegungsinformationen über den Probanden zu erfassen.

5. Übungsunterstützungssystem nach Anspruch 4, wobei eine Kommunikation zwischen der Übungsunterstützungsvorrichtung (3) und dem Bewegungssensor (2) entweder über kabelgebundene oder kabellose Kommunikationsmittel erfolgen kann.

6. Übungsunterstützungsverfahren, das eine Festlegung eines nächsten Übungsmenüs zur Verbesserung eines Zielkörpers auf der Grundlage von früheren Körperbewegungsinformationen über einen Probanden, die von einem Bewegungssensor (2) erfasst wurden, unterstützt, wobei das Übungsunterstützungsverfahren umfasst:
einen Schritt des Speicherns von Verteilungsinformationen über einen Aktivitätsumfang und eine Anzahl von Schritten von zumindest einer nach Aktivitätsintensität klassifizierten und vorab ausgewählten Kategorie in einer Gesundheitsgruppendaten-Speichereinheit (351), wobei die Verteilungsinformationen für die eine Kategorie vorab von einer Zielpersonengruppe für einen bestimmten Zeitraum erhalten werden;
einen Schritt des Berechnens des Aktivitätsumfangs und der Anzahl von Schritten für dieselbe Kategorie wie die ausgewählte Kategorie auf der Grundlage von Körperbewegungsinformationen über den Probanden, wobei die Bewegungsinformationen durch den Bewegungssensor (2) für denselben Zeitraum wie der bestimmte Zeitraum erfasst werden;
einen Schritt des Speicherns von Ergebnissen, die jeweils durch den Schritt des Berechnens berechnet wurden, in einer Messdaten-Speichereinheit (34); und
einen Schritt des Einlesens der in der Gesundheitsgruppendaten-Speichereinheit (351) gespeicherten Verteilungsinformationen in die Anzeigeeinheit (32), und
Anzeigen der Verteilungsinformationen mit einem Aktivitätsumfang und einer Anzahl von Schritten als Koordinatenachsen, nachdem die Ergebnisse in der Messdaten-Speichereinheit (34) gespeichert wurden,
Einlesen des Aktivitätsumfangs und der Anzahl von Schritten, die in der Messdaten-Speichereinheit (34) über den Probanden gespeichert sind, jeweils in die Anzeigeeinheit (32) als Informationen gradueller Positionsmarkierungen, und Anzeigen derselben mit den mitgeteilten Koordinatenachsen, denen die Verteilungsinformationen überlagert sind; und
einen Schritt des Empfangens der nächsten Festlegung des Übungsmenüs, während die Informationen gradueller Positionsmarkierungen den Verteilungsinformationen mit denselben Koordinatenachsen durch die Anzeigeeinheit (32) überlagert werden.

## Revendications

1. Dispositif de prise en charge d'exercice (3) qui prend en charge un réglage d'un menu d'exercice destiné à des améliorations en vue d'un corps cible, sur la base d'informations de mouvement corporel antérieures relatives à un sujet qui sont détectées par un capteur de mouvement (2), dans lequel le dispositif de prise en charge d'exercice (3) comprend :
une première unité de stockage de données de groupe en bonne santé (351) configurée pour stocker des informations de distribution relatives à une quantité d'activité et un nombre de pas d'au moins une catégorie qui sont classées par intensité d'activité et sélectionnées à l'avance, les informations de distribution pour ladite catégorie étant obtenues pour une période spécifiée à l'avance à partir d'un groupe de personnes cibles ;
une unité de traitement de quantité d'activité (312) configurée pour calculer la quantité d'activité et le nombre de pas d'une même catégorie que la catégorie sélectionnée, sur la base d'informations de mouvement corporel relatives au sujet, les informations de mouvement étant détectées par le capteur de mouvement (2) pour une même période que la période spécifiée, une unité de stockage de données de mesure (34) configurée pour stocker des résultats calculés à chaque fois par l'unité de traitement de quantité d'activité (312) ; et
une unité d'affichage (32) configurée pour afficher une image ;
un moyen de traitement de sortie (313), configuré pour lire les informations de distribution stockées dans l'unité de stockage de données de groupe en bonne santé (351) sur l'unité d'affichage (32), et pour afficher les informations de distribution avec une quantité d'activité et un nombre de pas sous la forme d'axes de coordonnées, une fois que l'unité de traitement de quantité d'activité (312) a stocké les résultats, et
pour lire la quantité d'activité et le nombre de pas stockées dans l'unité de stockage de données de mesure (34) qui sont relatifs au sujet à chaque fois sur l'unité d'affichage (32) sous la forme d'informations de marques de position graduelles, et pour les afficher avec les axes de coordonnées partagés sur lesquels les informations de distribution sont superposées ; et
une unité de traitement d'entrée (314) configurée pour recevoir un réglage ultérieur du menu d'exercice tandis que les informations de marques de position graduelles sont superposées sur les informations de distribution avec les mêmes axes de coordonnées par l'unité d'affichage (32).

2. Dispositif de prise en charge d'exercice (3) selon la revendication 1, dans lequel les informations de distribution sont des informations de marques de position de la quantité d'activité et du nombre de pas qui sont relatives à chaque sujet du groupe de personnes cibles.

3. Dispositif de prise en charge d'exercice (3) selon la revendication 1 ou la revendication 2, dans lequel la période spécifiée est une période qui est préréglée entre les périodes réveillée et endormie.

4. Système de prise en charge d'exercice comprenant le dispositif de prise en charge d'exercice selon l'une quelconque des revendications 1 à 3, et le capteur de mouvement (2) configuré pour détecter les informations de mouvement corporel relatives au sujet.

5. Système de prise en charge d'exercice selon la revendication 4, dans lequel la communication entre le dispositif de prise en charge d'exercice (3) et le capteur de mouvement (2) peut être communiquée par un moyen de communication soit filaire soit sans fil.

6. Procédé de prise en charge d'exercice qui prend en charge un réglage d'un menu d'exercice ultérieur destiné à des améliorations en vue d'un corps cible, sur la base d'informations de mouvement corporel antérieures relatives à un sujet qui sont détectées par un capteur de mouvement (2), dans lequel le procédé de prise en charge d'exercice comprend :
une étape destinée à stocker des informations de distribution relatives à une quantité d'activité et un nombre de pas d'au moins une catégorie qui sont classées par intensité d'activité et sélectionnées à l'avance dans une unité de stockage de données de groupe en bonne santé (351), les informations de distribution pour ladite catégorie étant obtenues pour une période spécifiée à l'avance à partir d'un groupe de personnes cibles ;
une étape destinée à calculer la quantité d'activité et le nombre de pas pour une même catégorie que la catégorie sélectionnée, sur la base d'informations de mouvement corporel relatives au sujet, les informations de mouvement étant détectées par le capteur de mouvement (2) pour une même période que la période spécifiée ;
une étape destinée à stocker des résultats calculés à chaque fois par l'étape de calcul dans une unité de stockage de données de mesure (34) ; et
une étape destinée à lire les informations de distribution stockées dans l'unité de stockage de données de groupe en bonne santé (351) sur l'unité d'affichage (32), et à afficher les informations de distribution avec une quantité d'activité et un nombre de pas sous la forme d'axes de coordonnées, une fois que les résultats ont été stockés dans l'unité de stockage de données de mesure (34),
à lire la quantité d'activité et le nombre de pas stockés dans l'unité de stockage de données de mesure (34) relatives au sujet à chaque fois sur l'unité d'affichage (32) sous la forme d'informations de marques de position graduelles, et à les afficher avec les axes de coordonnées partagés sur lesquels les informations de distribution sont superposées ; et
une étape destinée à recevoir un réglage ultérieur du menu d'exercice tandis que les informations de marques de position graduelles sont superposées sur les informations de distribution avec les mêmes axes de coordonnées par l'unité d'affichage (32).
